# EUROPÄISCHE PATENTSCHRIFT

(11) **EP 3 838 886 B1**
(45) Veröffentlichungstag und Bekanntmachung des Hinweises auf die Patenterteilung: **31.05.2023**
(21) Anmeldenummer: 19216884.7
(22) Anmeldetag: 17.12.2019
(51) Int. Cl.: C07C 67/38, C07C 69/608, C07C 29/149, C07C 31/27, C08K 5/00

(54) **ALKOXYCARBONYLIERUNG VON TRIVINYLCYCLOHEXAN**
ALKOXYCARBONYLATION OF TRIVINYL CYCLOHEXANE
ALCOXY-CARBONYLATION DU TRIVINYLCYCLOHEXANE

(43) Veröffentlichungstag der Anmeldung: 23.06.2021
(60) Teilanmeldung: 20193080.7
(73) Patentinhaber: Evonik Operations GmbH, 45128 Essen (DE)
(72) Erfinder: DONG, Kaiwu, 236800 Bo Zhou (CN); FRANKE, Robert, 45772 Marl (DE); JACKSTELL, Ralf, 18106 Rostock (DE); BELLER, Matthias, 18211 Ostseebad Nienhagen (DE)
(74) Vertreter: Evonik Patent Association

(56) Entgegenhaltungen:
- EP-A1- 3 272 733
- EP-A2- 0 495 175

## Beschreibung

Die vorliegende Erfindung betrifft ein Verfahren zur Alkoxycarbonylierung von Trivinylcyclohexan.

Die Alkoxycarbonylierung ethylenisch ungesättigter Verbindungen ist ein Prozess mit steigender Bedeutung. Unter einer Alkoxycarbonylierung versteht man die Umsetzung von ethylenisch ungesättigten Verbindungen, wie Olefinen, mit Kohlenmonoxid und Alkoholen in Gegenwart eines Metalls bzw. eines Metallkomplexes und eines Liganden zu den entsprechenden Estern:

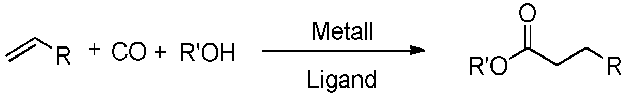

EP 0 495 175 A2 beschreibt carboxylgruppenhaltige Cyclohexanderivate, welche durch Hydrocarboxyalkylierung von 1,2,4-Trivinylcyclohexan erhalten werden. Bei der Umsetzung kommt ein Kobaltkatalysator zum Einsatz.

EP 3 272 733 A1 beschreibt ein Verfahren zur Alkoxycarbonylierung von Olefinen in einem Medium mit geringer Broenstedsäurenkonzentration.

Der vorliegenden Erfindung lag die Aufgabe zugrunde, ein Verfahren zur Alkoxycarbonylierung von Trivinylcyclohexan bereitzustellen. Mit diesem Verfahren sollen alle drei Vinylgruppen in Ester umgewandelt werden.

Des Weiteren soll ein schnell gelierender Weichmacher für Kunststoffe, insbesondere für PVC, bereitgestellt werden, welcher eine niedrige Verarbeitungstemperatur ermöglicht.

Diese Aufgabe wird gelöst durch ein Verfahren gemäß Anspruch 1.

Verfahren umfassend die Verfahrensschritte:
a) Vorlegen einer der Verbindungen (**i**), (**ii**), (**iii**), oder eines Gemisches aus mindestens zwei dieser Verbindungen: 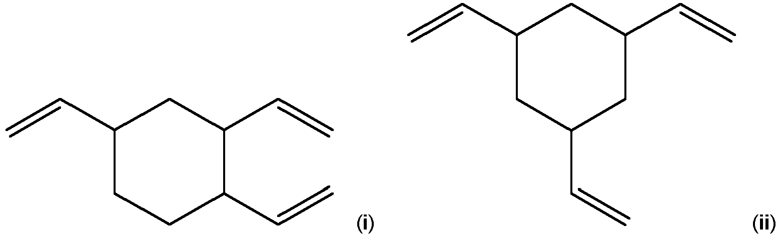 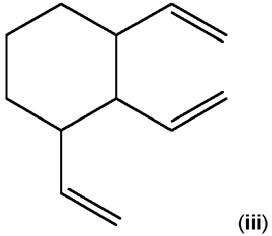
b) Zugabe des Liganden (L) und einer Verbindung, welche Pd umfasst, oder Zugabe eines Komplexes umfassend Pd und den Liganden (L): 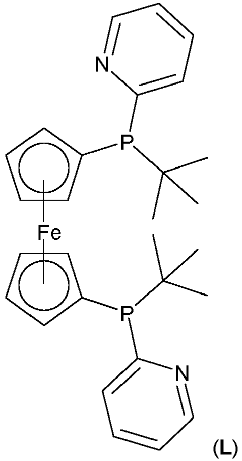
c) Zugabe eines Alkohols mit 1 bis 12 Kohlenstoff-Atomen;
d) Zuführen von CO;
e) Erwärmen des Reaktionsgemisches aus a) bis d), wobei die Verbindung / das Gemisch aus a) zu einem Triester umgesetzt wird;
f) Aufreinigung des Triesters;
g) Umsetzung des aufgereinigten Triesters mit NaOMe und H₂ zum Triol.

In einer Variante des Verfahrens wird im Verfahrensschritt a) die Verbindung (**i**) vorgelegt.

In einer Variante des Verfahrens wird im Verfahrensschritt a) die Verbindung (**ii**) vorgelegt.

In einer Variante des Verfahrens enthält der Alkohol in Verfahrensschritt c) neben dem Sauerstoff keine weiteren Heteroatome und keine Mehrfachbindungen.

In einer Variante des Verfahrens ist der Alkohol in Verfahrensschritt c) ausgewählt aus:
Methanol, Ethanol, "Butanol, Methylpropanol, "Pentanol, *^{iso}*Pentanol, 2-Methylbutanol, 3-Methylbutanol, "Hexanol, *^{iso}*Hexanol, "Heptanol, *^{iso}*Heptanol, "Octanol, *^{iso}*Octanol, 2-Ethylhexanol, "Nonanol, *^{iso}*Nonanol, "Decanol, *^{iso}*Decanol, 2-Propylheptanol.

In einer Variante des Verfahrens ist der Alkohol in Verfahrensschritt c) Methanol.

In einer Variante des Verfahrens erfolgt das Zuführen von CO in Verfahrensschritt d) bis zu einem Druck im Bereich von 20 bar bis 60 bar.

In einer Variante des Verfahrens erfolgt das Zuführen von CO in Verfahrensschritt d) bis zu einem Druck im Bereich von 30 bar bis 50 bar.

In einer Variante des Verfahrens erfolgt das Erwärmen in Verfahrensschritt e) auf eine Temperatur im Bereich von 90 °C bis 130 °C.

In einer Variante des Verfahrens erfolgt das Erwärmen in Verfahrensschritt e) auf eine Temperatur im Bereich von 100 °C bis 120 °C.

In einer Variante des Verfahrens wird die Umsetzung in Verfahrensschritt g) mit Ru-MACHO-BH katalysiert.

In einer Variante des Verfahrens erfolgt die Umsetzung in Verfahrensschritt g) mit einem H₂-Druck im Bereich von 30 bar bis 70 bar.

In einer Variante des Verfahrens erfolgt die Umsetzung in Verfahrensschritt g) mit einem H₂-Druck im Bereich von 40 bar bis 60 bar.

In einer Variante des Verfahrens erfolgt die Umsetzung in Verfahrensschritt g) bei einer Temperatur im Bereich von 80 °C bis 120°C.

In einer Variante des Verfahrens erfolgt die Umsetzung in Verfahrensschritt g) bei einer Temperatur im Bereich von 90 °C bis 110 °C.

Im Folgenden soll die Erfindung anhand eines Ausführungsbeispiels näher erläutert werden.

### Synthese 3,3',3"-(Cyclohexan-1,2,4-triyl)tripropionsäuretrimethylester (1) ("Triester")

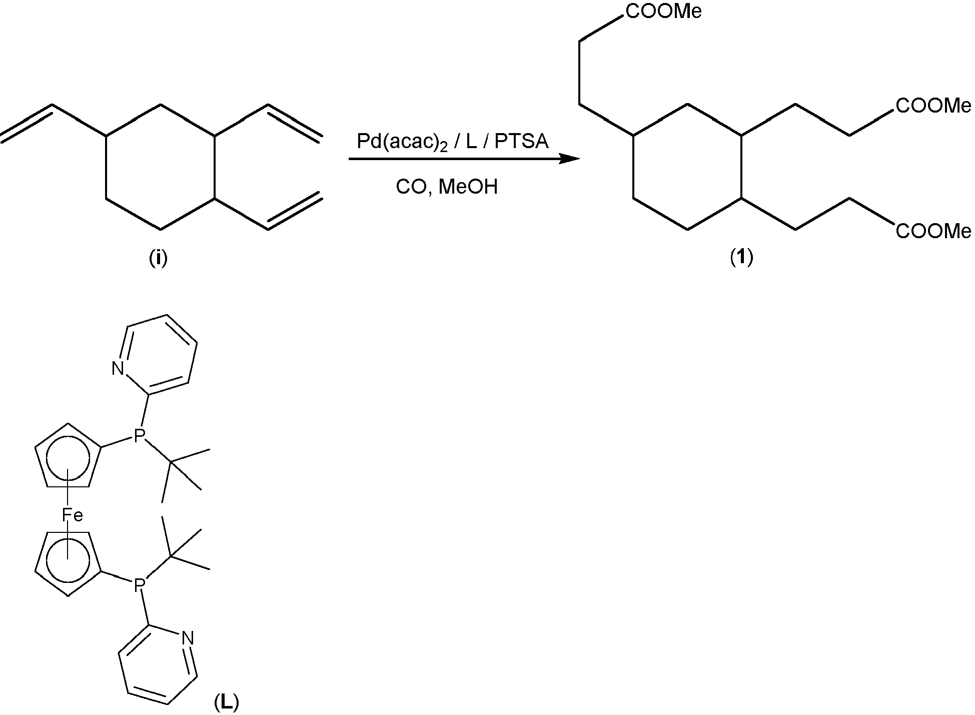

[Pd(acac)₂] (15,2 mg, 0,1 mol%), **L** (103 mg, 0,4 mol%) und p-Toluolsulfonsäure (PTSA) (143 mg, 1,5 mol%) wurden unter einer Argonatmosphäre in einen 100-ml-Stahlautoklaven gegeben. Dann wurden MeOH (30 ml) und Trivinylcyclohexan (i) (8,1 g, 50 mmol) mittels einer Spritze injiziert. Der Autoklav wurde dreimal mit CO gespült und anschließend mit einem CO-Druck von 40 bar beaufschlagt. Die Reaktion erfolgte über 10 h bei 110 °C. Danach wurde der Autoklav auf Raumtemperatur abgekühlt und entspannt. Das gewünschte Produkt wurde durch Destillation (165 °C bei 10⁻³ bar) aufgereinigt und mit ¹H-, ¹³C-NMR und HR-MS charakterisiert (15,6 g, 91% Ausbeute).

¹H-NMR (300 MHz, C₆D₆) δ = 3,39-3,37 (m, 9H), 2,24-1,86 (m, 7H), 1,48-0,27 (m, 14 H).

¹³C-NMR (75 MHz, C₆D₆) δ = 173,68-173,54 (m), 51,04, 40,60-40,47 (m), 38,24, 38,14, 37,51, 37,07, 36,54, 36,10, 35,52, 35,14, 33,87, 32,70, 32,55, 32,51, 32,38, 32,29, 32,23, 32,08, 31,97, 31,86, 31,76, 31,68, 31,63, 31,43, 30,98, 30,79, 30,75, 29,31, 28,52, 28,47, 28,34, 28,13, 28,11, 27,13, 26,58, 25,12, 20,79, 19,74.

MS (EI): 311 (13,40), 293 (3,65), 269 (75,76), 237 (60,40), 219 (25,13), 205 (100), 191 (17,62), 177 (14,83), 145 (24,59).

HR-MS (ESI): Berechnet C₁₈H₃₀O₆ [M + H]⁺: 343,21152, Gefunden: 343,21113.

### Synthese 3,3',3"-(Cyclohexan-1,2,4-triyl)tris(propan-1-ol) (2) ("Triol")

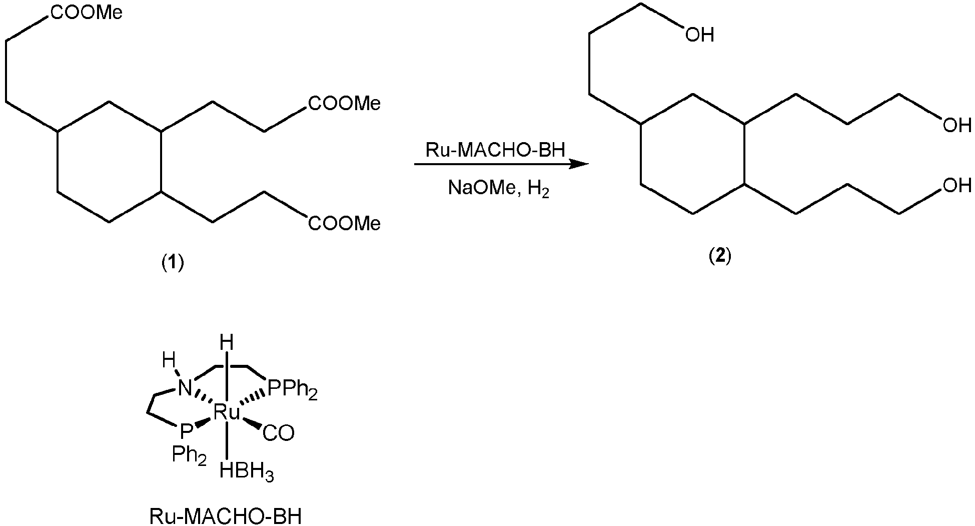

Ru-MACHO-BH (59 mg, 2 mol%) und NaOMe (27 mg, 10 mol%) wurden unter einer Argonatmosphäre in einen 25 ml-Stahlautoklaven gegeben. Dann wurden MeOH (8 ml) und der Triester (1) (1,93 g, 5,67 mmol) mittels Spritze injiziert. Der Autoklav wurde dreimal mit H₂ gespült und anschließend mit einem H₂-Druck von 50 bar beaufschlagt. Die Reaktion erfolgte über 10 h bei 100 °C. Der Autoklav wurde danach auf Raumtemperatur abgekühlt und entspannt. Das gewünschte Produkt wurde durch Filtration über Kieselgel aufgereinigt und durch ¹H-, ¹³C-NMR und HR-MS charakterisiert (1,4 g, 96% Ausbeute).

¹H-NMR (400 MHz, CD₃OD) δ = 3,32-3,30 (m, 6H), 1,85-1,77 (m, 2H), 1,63-0,62 (m, 19H). ¹³C-NMR (100 MHz, CD₃OD) δ = 63,53-63,29 (m), 42,69, 42,53, 42,39, 42,31, 40,34, 40,24, 39,28, 38,89, 38,12, 38,02, 37,58, 37,27, 35,89, 34,78, 34,72, 34,53, 34,37, 33,17, 31,52, 31,50, 31,40, 31,38, 31,05, 30,60, 30,48, 30,41, 30,32, 30,24, 30,17, 28,77, 28,22, 26,61, 22,53, 21,45. MS (EI): 222 (1,06), 194 (1,14), 181 (2,96), 163 (11,54), 135 (9,81), 121 (17,04), 107 (15,25), 93 (32,69).

HR-MS (ESI): Berechnet C₁₅H₃₀O₃ [M + H]⁺: 259,22677, Gefunden: 259,2269.

### Herstellung von Plastisolen

Es wurden PVC-Plastisole hergestellt, wie sie beispielsweise zur Fertigung von Deckstrichfilmen für Fußbodenbeläge verwendet werden. Die Angaben in der Plastisolrezeptur ist jeweils in Massenanteilen. Die Rezeptur der Polymerzusammensetzung ist in Tabelle 1 aufgelistet.

**Tabelle 1: Plastisolrezeptur**

| | phr |
|---|---|
| PVC (Vestolit B 7021 - Ultra; Fa. Vestolit) | 100 |
| Weichmacher | 50 |
| epoxidiertes Sojabohnenöl als Co-Stabilisator (Drapex 39, Fa. Galata) | 3 |
| Thermostabilisator auf Ca/Zn-Basis (Reagens CLX/759/6PF) | 2 |

| | |
|---|---|
| Angaben in phr (phr = parts per hundred parts resin) | |

Zuerst werden die flüssigen und dann die pulverförmigen Bestandteile in einen PE-Becher eingewogen. Von Hand wird die Mischung mit einem Salbenspatel so eingerührt, dass kein unbenetztes Pulver mehr vorhanden ist. Der Mischbecher wird dann in die Klemmvorrichtung eines Dissolverrührers eingespannt. Nach Einschalten des Rührers wird die Drehzahl langsam auf ca. 2000 UpM erhöht. Währenddessen wird das Plastisol vorsichtig entlüftet, der Druck muss 20 mbar unterschreiten.

Sobald das Plastisol eine Temperatur von ca. 30 °C erreicht hat, wird die Drehzahl auf ca. 350 UpM gesenkt. Ab jetzt wird das Plastisol für 9 Minuten bei dieser Drehzahl und einem Druck unterhalb von 20 mbar entlüftet. Damit war sichergestellt, dass die Homogenisierung des Plastisols bei einem definierten Energieeintrag erreicht wurde. Danach wird das Plastisol sofort für weitere Untersuchungen in einem Klimaschrank auf 25,0 °C temperiert.

### Gelierverhalten der Plastisole

Die Untersuchung des Gelierverhaltens der Pasten wurde mit einem Physica MCR 101 im Oszillationsmodus mit einem Platte-Platte Messsystem (PP25), welches schubspannungsgesteuert betrieben wurde, vorgenommen. Eine zusätzliche Temperierhaube wurde an das Gerät angeschlossen, um eine homogene Wärmeverteilung und eine gleichmäßige Probentemperatur zu erreichen.

Folgende Parameter wurden eingestellt:

| Modus: | Temperatur-Gradient |
|---|---|
| Start-Temperatur: | 25 °C |
| End-Temperatur: | 180 °C |
| Heiz/Kühlrate: | 5 °C/min |
| Oszillations-Frequenz: | 4-0,1 Hz Rampe logarithmisch |
| Kreisfrequenz Omega: | 10 1/s |
| Anzahl Messpunkte: | 63 |
| Messpunktdauer: | 0,5 min |
| Automatische Spaltnachführung F: | 0 N |
| Konstante Messpunktdauer | |
| Spaltweite | 0,5 mm |

### Durchführung der Messung

Auf die untere Messsystemplatte werden mit dem Spatel einige Gramm der zu messenden Paste luftblasenfrei aufgetragen. Dabei wird darauf geachtet, dass nach dem Zusammenfahren des Messsystems etwas Paste gleichmäßig aus dem Messsystem herausquellen kann (nicht mehr als 6 mm rundum). Anschließend wird die Temperierhaube über der Probe positioniert und die Messung gestartet. Bestimmt wird die komplexe Viskosität der Paste nach 24 Stunden (Lagerung der Paste bei 25 °C in einem Temperierschrank der Fa. Memmert) in Abhängigkeit von der Temperatur.

Als Maß für die Gelierung wird ein deutlicher Anstieg der komplexen Viskosität betrachtet. Als Vergleichswert wird daher die Temperatur bei Erreichen einer Pastenviskosität von 1000 Pas verwendet.

Der Versuche wurde mit drei Vergleichsplastisolen wiederholt, bei welchen jeweils ein anderer Weichmacher verwendet wurde.

**Tabelle 2: Gelierung der Plastisole nach 24 Stunden, Temperatur in °C bei Erreichen einer Pastenviskosität von 10³ Pa·s**

| Versuch | Weichmacher | Geliertemperatur [°C] |
|---|---|---|
| 1* | 3,3',3"-(Cyclohexan-1,2,4-triyl)tripropionsäuretrimethylester **(1)** | 65 |
| 2 | Diisononylphthalat (DINP), VESTINOL 9 von Evonik Performance Materials GmbH | 83 |
| 3 | 1,2-Cyclohexandicarbonsäurediisononylester (DINCH), ELATUR CH von Evonik Performance Materials GmbH | 101 |
| 4 | Diisopentylterephthalat (DPT), ELATUR DPT von Evonik Performance Materials GmbH | 70 |

| | | |
|---|---|---|
| * Versuch mit erfindungsgemäßer Verbindung | | |

Der angestrebte Wert von 1000 Pa*s konnte mit der erfindungsgemäßen Verbindung (1) bereits bei 65 °C erreicht werden. Solch niedrige Geliertemperaturen sind vorteilhaft für den Verarbeitungsprozess. Sie ermöglichen eine Plastisolverarbeitung bei niedrigeren Temperaturen.

## Patentansprüche

1. Verfahren umfassend die Verfahrensschritte:
a) Vorlegen einer der Verbindungen (i), (ii), (iii), oder eines Gemisches aus mindestens zwei dieser Verbindungen: 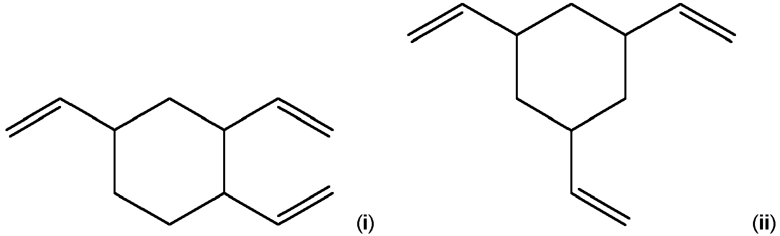 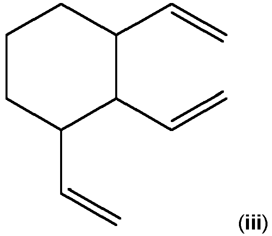
b) Zugabe des Liganden (L) und einer Verbindung, welche Pd umfasst, oder Zugabe eines Komplexes umfassend Pd und den Liganden (L): 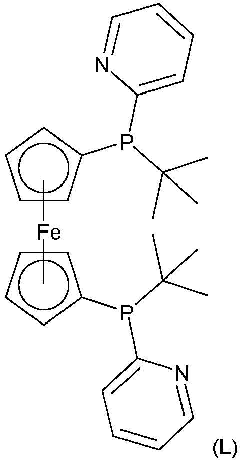
c) Zugabe eines Alkohols mit 1 bis 12 Kohlenstoff-Atomen;
d) Zuführen von CO;
e) Erwärmen des Reaktionsgemisches aus a) bis d), wobei die Verbindung / das Gemisch aus a) zu einem Triester umgesetzt wird;
f) Aufreinigung des Triesters;
g) Umsetzung des aufgereinigten Triesters mit NaOMe und H₂ zum Triol.

2. Verfahren nach Anspruch 1,
wobei im Verfahrensschritt a) die Verbindung (**i**) vorgelegt wird.

3. Verfahren nach Anspruch 1,
wobei im Verfahrensschritt a) die Verbindung (**ii**) vorgelegt wird.

4. Verfahren nach einem der Ansprüche 1 bis 3,
wobei der Alkohol in Verfahrensschritt c) neben dem Sauerstoff keine weiteren Heteroatome und keine Mehrfachbindungen enthält.

5. Verfahren nach einem der Ansprüche 1 bis 4,
wobei der Alkohol in Verfahrensschritt c) ausgewählt ist aus:
Methanol, Ethanol, "Butanol, Methylpropanol, "Pentanol, *^{iso}*Pentanol, 2-Methylbutanol, 3-Methylbutanol, "Hexanol, *^{iso}*Hexanol, "Heptanol, *^{iso}*Heptanol, "Octanol, *^{iso}*Octanol, 2-Ethylhexanol, "Nonanol, *^{iso}*Nonanol, "Decanol, *^{iso}*Decanol, 2-Propylheptanol.

6. Verfahren nach einem der Ansprüche 1 bis 5,
wobei der Alkohol in Verfahrensschritt c) Methanol ist.

7. Verfahren nach einem der Ansprüche 1 bis 6,
wobei das Zuführen von CO in Verfahrensschritt d) bis zu einem Druck im Bereich von 20 bar bis 60 bar erfolgt.

8. Verfahren nach einem der Ansprüche 1 bis 7,
wobei das Erwärmen in Verfahrensschritt e) auf eine Temperatur im Bereich von 90 °C bis 130 °C erfolgt.

9. Verfahren nach Anspruch 1,
wobei die Umsetzung in Verfahrensschritt g) mit Ru-MACHO-BH katalysiert wird.

10. Verfahren nach einem der Ansprüche 1 oder 9,
wobei die Umsetzung in Verfahrensschritt g) mit einem H₂-Druck im Bereich von 30 bar bis 70 bar erfolgt.

11. Verfahren nach einem der Ansprüche 1 bis 10,
wobei die Umsetzung in Verfahrensschritt g) bei einer Temperatur im Bereich von 80 °C bis 120 °C erfolgt.

## Claims

1. Process comprising the process steps of:
a) initially charging one of the compounds (i), (ii), (iii), or a mixture of at least two of these compounds: 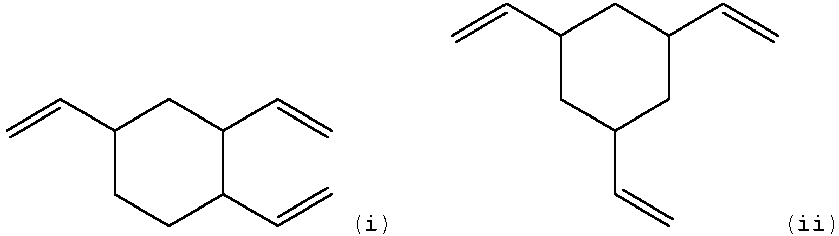 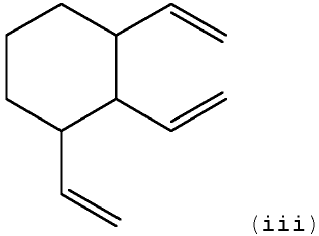
b) adding the ligand (L) and a compound comprising Pd, or adding a complex comprising Pd and the ligand (L): 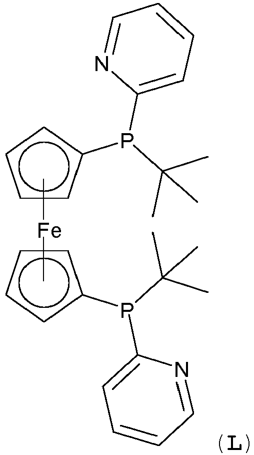
c) adding an alcohol having 1 to 12 carbon atoms;
d) feeding in CO;
e) heating the reaction mixture of a) to d), wherein the compound/the mixture of a) is converted to a triester;
f) purifying the triester;
g) reacting the purified triester with NaOMe and H₂ to give the triol.

2. Process according to Claim 1,
wherein the compound (**i**) is initially charged in process step a).

3. Process according to Claim 1,
wherein the compound (**ii**) is initially charged in process step a).

4. Process according to any of Claims 1 to 3,
wherein the alcohol in process step c), besides the oxygen, does not comprise any further heteroatoms and contains no multiple bonds.

5. Process according to any of Claims 1 to 4,
wherein the alcohol in process step c) is selected from: methanol, ethanol, *ⁿ*butanol, methylpropanol, *ⁿ*pentanol, *^{iso}*pentanol, 2-methylbutanol, 3-methylbutanol, *ⁿ*hexanol, *^{iso}*hexanol, *ⁿ*heptanol, *^{iso}*heptanol, *ⁿ*octanol, *^{iso}*octanol, 2-ethylhexanol, *ⁿ*nonanol, *^{iso}*nonanol, *ⁿ*decanol, *^{iso}*decanol, 2-propylheptanol.

6. Process according to any of Claims 1 to 5,
wherein the alcohol in process step c) is methanol.

7. Process according to any of Claims 1 to 6,
wherein CO is fed in in process step d) up to a pressure in the range from 20 bar to 60 bar.

8. Process according to any of Claims 1 to 7,
wherein the heating in process step e) is carried out at a temperature in the range from 90°C to 130°C.

9. Process according to Claim 1,
wherein the reaction in process step g) is catalysed by Ru-MACHO-BH.

10. Process according to either of Claims 1 or 9,
wherein the reaction in process step g) is carried out at a H₂ pressure in the range of 30 bar to 70 bar.

11. Process according to any of Claims 1 to 10,
wherein the reaction in process step g) is carried out at a temperature in the range of 80°C to 120°C.

## Revendications

1. Procédé comprenant les étapes de procédé :
a) chargement d'un des composés (i), (ii) (iii), ou d'un mélange d'au moins deux de ces composés : 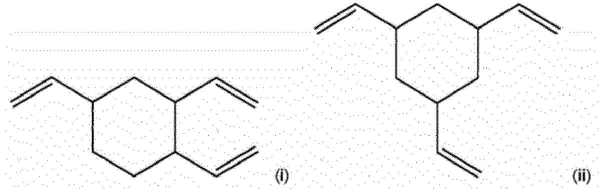 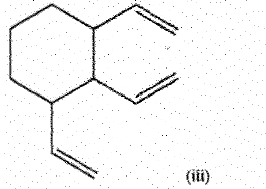
b) ajout du ligand (L) et d'un composé qui comprend du Pd, ou ajout d'un complexe comprenant du Pd et le ligand (L) : 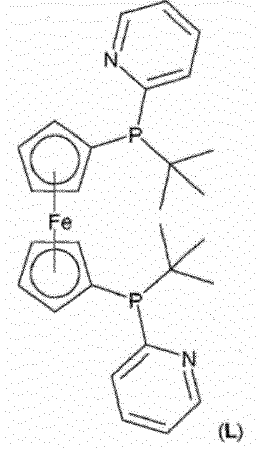
c) ajout d'un alcool comportant 1 à 12 atomes de carbone ;
d) introduction de CO ;
e) chauffage du mélange réactionnel de a) à d), le composé/le mélange de a) étant transformé en un triester ;
f) purification du triester ;
g) transformation avec NaOMe et H₂ du triester purifié pour donner le triol.

2. Procédé selon la revendication 1, le composé (i) étant chargé dans l'étape de procédé a).

3. Procédé selon la revendication 1, le composé (ii) étant chargé dans l'étape de procédé a).

4. Procédé selon l'une quelconque des revendications 1 à 3, l'alcool dans l'étape de procédé c) ne contenant, outre l'oxygène, aucun autre hétéroatome et aucune double liaison.

5. Procédé selon l'une quelconque des revendications 1 à 4, l'alcool dans l'étape de procédé c) étant choisi parmi :
méthanol, éthanol, *ⁿ*butanol, méthylpropanol, *ⁿ*pentanol, *^{iso}*pentanol, 2-méthylbutanol, 3-méthylbutanol, *ⁿ*hexanol, *^{iso}*hexanol, *ⁿ*heptanol, *^{iso}*heptanol, *ⁿ*octanol, *^{iso}*octanol, 2-éthylhexanol, *ⁿ*nonanol, *^{iso}*nonanol, *ⁿ*décanol, *^{iso}*décanol, 2-propylheptanol.

6. Procédé selon l'une quelconque des revendications 1 à 5, l'alcool dans l'étape de procédé c) étant le méthanol.

7. Procédé selon l'une quelconque des revendications 1 à 6, l'introduction de CO dans l'étape de procédé d) étant réalisée jusqu'à une pression dans la plage de 20 bars à 60 bars.

8. Procédé selon l'une quelconque des revendications 1 à 7, le chauffage dans l'étape de procédé e) étant réalisé à une température dans la plage de 90 °C à 130 °C.

9. Procédé selon la revendication 1, la transformation dans l'étape de procédé g) étant catalysée par Ru-MACHO-BH.

10. Procédé selon l'une quelconque des revendications 1 ou 9, la transformation dans l'étape de procédé g) étant réalisée avec une pression de H₂ dans la plage de 30 bars à 70 bars.

11. Procédé selon l'une quelconque des revendications 1 à 10, la transformation dans l'étape de procédé g) étant réalisée à une température dans la plage de 80 °C à 120 °C.
